# EUROPEAN PATENT SPECIFICATION

(11) **EP 2 220 210 B1**
(45) Date of publication and mention of the grant of the patent: **12.08.2015**
(21) Application number: 08855553.7
(22) Date of filing: 21.11.2008
(51) Int. Cl.: C12N 1/20, A23L 1/30, A61K 35/74, C12R 1/25

(54) **STRAINS OF LACTOBACILLUS PLANTARUM AS PROBIOTICS WITH IMMUNOMODULATORY SPECIFIC EFFECT**
LACTOBACILLUS PLANTARUM-STÄMME ALS PROBIOTIKA MIT IMMUNOMODULATORISCHEM SPEZIFISCHEM EFFEKT
SOUCHES DE<I>LACTOBACILLUS PLANTARUM</I>EN TANT QUE PROBIOTIQUE AVEC EFFET IMMUNOMODULATEUR SPÉCIFIQUE

(30) Priority: 29.11.2007 EP 07121817
(43) Date of publication of application: 25.08.2010
(73) Proprietor: CARINSA. Creaciones Aromáticas Industriales, S.A., 08192 Sant Quirze des Vallés (Barcelona) (ES)
(72) Inventor: MARTÍNEZ CHAMORRO, Vanesa, E-08205 Sabadell (ES); LÓPEZ GONZÁLEZ, Quiro, E-08205 Sabadell (ES); GASULL, Miquel Àngel, E-08006 Barcelona (ES); CUÑÉ CASTELLANA, Jordi, E-25210 Gissona (ES); ESPADALER MAZO, Jordi, E-17003 Girona (ES); CABRÉ GELADA, Eduard, E-08015 Barcelona (ES); MAÑÉ ALMERO, Josep, E-08917 Badalona (ES); CALVO TORRAS, Maria de los Ángeles, E-08015 Barcelona (ES); BONACHERA SIERRA, Miquel Àngel, E-17130 L'escala (ES); AUDIVERT BRUGUÉ, Sergi, E-17841 Sarrià De Ter (ES)
(74) Representative: ZBM Patents - Zea, Barlocci & Markvardsen
(86) International application number: PCT/EP2008/065950
(87) International publication number: WO 2009/068474

(56) References cited:
- EP-A- 1 661 983
- WO-A-2007/003917
- PARK Y-S ET AL: "Isolation and characterization of lactic acid bacteria from feces of newborn baby and from dongchimi" JOURNAL OF AGRICULTURAL AND FOOD CHEMISTRY, AMERICAN CHEMICAL SOCIETY. WASHINGTON, US, vol. 50, no. 9, 24 April 2002 (2002-04-24), pages 2531-2536, XP002232454 ISSN: 0021-8561
- HERIAS M V ET AL: "Immunomodulatory effects on lactobacillus plantarum colonizing the intestine of gnotobiotic rats" CLINICAL AND EXPERIMENTAL IMMUNOLOGY, OXFORD, GB, vol. 116, no. 2, 1 January 1999 (1999-01-01), pages 283-290, XP002904113 ISSN: 0009-9104
- JACOBSEN C N ET AL: "Screening of probiotic activities of forty-seven strains of Lactobacillus spp. by in vitro techniques and evaluation of the colonization ability of five selected strains in humans" APPLIED AND ENVIRONMENTAL MICROBIOLOGY, WASHINGTON,DC, vol. 65, no. 11, 1 November 1999 (1999-11-01), pages 4949-4956, XP002232455 ISSN: 0099-2240
- OCANA VIRGINIA S ET AL: "Selection of vaginal H2O2-generating Lactobacillus species for probiotic use" CURRENT MICROBIOLOGY, vol. 38, no. 5, May 1999 (1999-05), pages 279-284, XP002479825 ISSN: 0343-8651

## Description

The present invention relates to the fields of medicine, microbiology and food technology and particularly, to novel strains of Lactobacillus plantarum for using as probiotics to benefit human health.

### BACKGROUND ART

Deficiencies in the functioning of the immune system may be a consequence of old age, stress, infectious illness (e.g. acquired immune deficiency syndrome) and malnutrition. In many Western countries, the elderly represent a growing proportion of the population, and one for which health care expenditure is disproportionately high. Many of the health issues faced by the elderly are represented by an increased age-dependent risk of morbidity, due to an increased susceptibility to infectious and noninfectious diseases. The immune defense system in particular is known to be adversely affected by the aging process, and there is strong evidence that a poorly functioning immune system can contribute to decreased disease resistance and reduced life expectancy in the elderly. Immunosenescence is particularly characterized by suboptimal function of the cellular immune system. The most well-characterized age-related changes involve thymic (T) lymphocytes, including a decrease in the numbers of mature CD3+ T cells in peripheral circulation, a decrease in the pool of naïve (CD45RA+) T cells, a predisposition toward T helper 2 phenotype expression, and a poor capacity of peripheral blood T cells to proliferate and secrete interleukin 2 or to contribute to effective delayed-type hypersensitivity responses in vivo. Immunosenescence can also be expressed by changes in the function or proportions of leukocytes that contribute to innate immunity, such as phagocytes and natural killer (NK) cells. Up to 70% of subjects older than 70 years have low NK cell-mediated cytotoxicity responses against K562 tumor cells compared with 40% of young adults. A decline in innate immune cell function is generally considered to be a contributing factor to decreased immunity in the elderly. Moreover, because both NK cells and phagocytes (particularly monocytes) secrete many immunoregulatory cytokines, their potentially diminished function in elderly individuals may have important downstream effects on immune events in the integrated immune system, such as lymphocyte activation and differentiation (cf. H.S. Gill et al., "Dietary probiotic supplementation enhances natural killer cell activity in the elderly: an investigation of age-related immunological changes", Journal of Clinical Immunology 2001, vol. 21, pp. 264-271). Accordingly, interventions that can combat immunosenescence by restoring cellular immune function are highly desirable.

The findings that these immunological deficiencies could be overcome by modulating the immune system have stimulated a search for natural and chemical agents and/or products with immunomodulatory properties. Unfortunately, the use of many currently available immunostimulatory products is associated with deleterious side-effects (cf. A.K. Nussler and A.W. Thomson, "Immunomodulatory agents in the laboratory and clinic", Parasitology 1992, vol. 105, S5-S23). The development of natural food products with immunoenhancing properties, that are free of side-effects, would therefore be of significant benefit to population groups with impaired immune function. Therefore, an attractive means of restoring immune function is dietary intervention. Previous research in the elderly showed that dietary supplementation with micronutrients (e.g. vitamins A, C and E; β-carotene) enhanced some aspects of cellular immunity, including the proportion of circulating T lymphocyte subsets and the in vitro activity of blood-derived NK cells. Another dietary regimen that could benefit the elderly is probiotic supplementation with immunostimulating strains of lactic acid bacteria (LAB).

Probiotics are live non-pathogenic microorganisms usually found in the gastrointestinal tract that, when administered in adequate amounts, exert health benefits. Specific conditions for which probiotics are used include increasing in the resistance to infection, prevention and treatment of antibiotic-associated diarrhea and other diarrhea illnesses, genitourinary tract infection prophylaxis, increasing immune function, decreasing cholesterol and lipid levels, increasing colonization resistance to harmful microbiota, and various immune disorders including atopic dermatitis and food allergy. Certain well-defined lactic acid bacteria strains have shown to be potent enhancers of immunity and to offer clinical benefits to defined groups. For example, Lactobacillus rhamnosus strain GG has been used successfully as a dietary immunomodulatory in pediatric care. In contrast, however, the ability of defined probiotic lactic acid bacteria strains to modulate immunity in the elderly has not been well-established (H. Gill et al., "Enhancement of immunity in the elderly by dietary supplementation with the probiotic Bifidobacterium lactis HN019", Am. J. Clin. Nutr. 2001, vol. 74, pp. 833-9).

These findings have stimulated interest in finding new strains of lactobacilli and bifidobacteria that are able to enhance immunity. Therefore, it is desirable to provide new agents with probiotic properties to benefit health, including geriatric health.

### SUMMARY OF THE INVENTION

The inventors provide two new strains of Lactobacillus plantarum suitable as probiotics as a result of extensive studies of different bacterial strains isolated from feces and saliva from humans fed with a vegetables diet. The strains of the invention were deposited on 18.09.2007 in the Colección Española de Cultivos Tipo (CECT) under the Accession Numbers CECT 7315 and 7316.

The strains CECT 7315 and 7316 were those from feces samples which grew in a MRS medium supplemented with 0.05 % w/v HCl, 100 µg/l Novobiocin (Sigma), 5 µg/ml nystatin 5 µg/ml cyclohexamide (Sigma), 1 mg/l ampicilin, 10 µg/ml vancomycin and incubated at 30 °C under anaerobic conditions and pH 6.4. Gram staining showed a clear Gram+ staining, as well as bacilli morphology, non-spore-forming.

There is a diversity of Lactobacillus plantarum strains under study and presently marketed as probiotics (cf. M.C. de Vries et al., "Lactobacillus plantarum-survival, functional and potential probiotic properties in the human intestinal tract", International Dairy Journal 2006, vol. 16, pp. 1018-1028). It is important to note however, that a large variation exists in the ability to enhance immune function and only a few strains with well-established immunity-enhancing effects have been identified to date. It has been found that the presence of immunomodulatory properties of probiotics and the pattern of immunomodulation depend on the bacterial species involved, the individual strain, the use of viable or killed bacteria, and the bacterial dose.

As it is illustrated below, the results of a clinical study with healthy elderly humans, show that supplementation with the two strains of the invention is effective in enhancing both natural and acquired immunity in humans.

Particularly, CD4+ cells and CD8+ cells showed a significant increase in activity as indicated by CD25 expression. B lymphocytes (CD19+) and Natural Killer cells (CD59+) showed a significant increase in number and antigen presenting cells (HLA-DR+) were stimulated as well. Further, a significant decrease in pro-inflammatory cytokine release (TGF-β) was observed. This result, together with the cellular immunity retrieval, may indicate that the immune regulation moves from unspecific (cytoquines) towards more specific (cellular). Furthermore, the strains have demonstrated that are particularly useful as probiotics in food industry. The strains have proven that:
- have a grow efficiency suitable to be industrially produced and economically viable;
- are capable of fermenting milk to form yogurt and none present unpleasant odors;
- survive the conditions of the gastrointestinal environment of mammals (lysozyme, acid environment, bile salts and oxygen peroxide);
- adhere to the intestinal epithelium, which allows strains to remain at least transiently in the intestinal tract and to exert their probiotic effects; they show a better adhesion capability compared with other commercial strains;
- have no negative effects on animal behavior and well-being in an acute toxicity study with rats;
- are safe since they do not lead to an increase in translocation of lactic bacteria nor do they facilitate enterobacterial translocation;
- benefit the intestinal microbiota balance due to their capacity to inhibit the growth of pathogenic strains; and
- secrete one or more soluble factors responsible of the antibacterial activity.

Thus, in one aspect, the present invention relates to a strain of Lactobacillus plantarum deposited in the Colección Española de Cultivos Tipo (CECT) under the accession number CECT 7315.

In a second aspect, the present invention relates a strain of Lactobacillus plantarum deposited in the Colección Española de Cultivos Tipo (CECT) under the accession number CECT 7316.

In a third aspect, the present invention relates to a bacterial culture which comprises the strain 7315 or the strain 7316 or a mixture thereof.

As it is illustrated below in a non-restricted way, it has been found that the Lactobacillus plantarum strains of the present invention are useful in the food industry and particularly as probiotics.

Thus, in a fourth aspect, the present invention relates to a product which comprises an effective amount of the strain 7315 or of the strain 7316 or of a mixture thereof.

In a fifth aspect, the present invention relates to an edible product which comprises a nutritionally effective amount of the strain 7315 or of the strain 7316 or of a mixture thereof, together with appropriate amounts of other edible ingredients.

In a sixth aspect, the present invention relates to a pharmaceutical composition which comprises a therapeutically effective amount of the strain 7315 or of the strain 7316 or of a mixture thereof, together with appropriate amounts of pharmaceutical acceptable excipients and/or carriers.

In a seventh aspect, the present invention relates to the use of the strains of the invention or a mixture thereof for the manufacture of an edible product.

In an eighth aspect, the present invention relates to the use of the strains of the invention or of a mixture thereof for the manufacture of a medicament for enhancing the immune system of an animal including a human. The invention may alternatively be formulated as a method for enhancing the immune system of an animal including a human, comprising administering to said animal in need thereof an effective amount of the strain 7315 or of the strain 7316 or of a mixture thereof. The use of the strains of the invention may be beneficial in the sense of being usable prophylactically for a normal healthy individual, preventing the onset of a poorly functioning immune state or before a disease has developed. The administration of the strains of the invention may be beneficial also in the therapeutic treatment of states which present a deregulation of the immune system (e.g. immunosenescence) sometimes associated to a disease. The strains can be administered as treatment or as a complement of another treatment.

In a ninth aspect, the present invention relates to a process for the preparation of an edible product, comprising cultivating the strain 7315 or the strain 7316 or a mixture thereof, in a suitable medium (e.g. milk, whey protein, technical medium). The bacteria can be added to a food product during the manufacturing process thereof, or to a finished product. Appropriate amounts of other edible ingredients could be added.

These and other objects of the present invention will be further described in the detailed description section that follows, and they are not intended to be limiting of the present invention. Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention.

### BRIEF DESCRIPTION OF THE DRAWINGS

FIG.1 shows the growth curves of F35 (FIG. 1A) and F47 (FIG. 1B). log cfu/ml is plotted against T (time, in h).
FIG. 2 shows the phylogenetic classification of F35 and F47 strains based on their 16S sequence, with regard to the Lactobacillus strains most generically characterized.
FIG. 3 shows the genomic macro-restriction patterns with enzymes Sfi-I (upper) and Sma-I (lower). M, marker; 1: 299v; 2: F35; 3: F47. (*) means bands which are different between patterns.
FIG. 4 shows the % of adhesion of strains F35 and F47 vs. two commercial strains of Lactobacillus reuteri and Lactobacillus rhamnosus.
FIG. 5 shows the results of intestinal transit regulation in the clinical study. The dark part of the graphic corresponds to the individuals with 0-3.5 defecations/week and the part without color, with 3.5-7 defecations/week. "w" means week. FIG. 5A corresponds to placebo group (n=15). From basal to 24 weeks were no statistically significant differences. FIG. 5B corresponds to groups administered with the probiotics, and p<0.05.

### DETAILED DESCRIPTION OF PARTICULAR EMBODIMENTS

A detailed characterization of the strains of the invention, their properties and products derived from, are described in sections further on.

As it is used in the art, the term "probiotic" refers to bacteria in the context of dietary supplements. Generally, the term probiotic refers potentially beneficial bacteria or yeast, with lactic acid bacteria as the most common microbes used. The term "effective amount" as used herein, means an amount of an active ingredient high enough to deliver the desired benefit, but low enough to avoid serious side effects within the scope of medical judgment. The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. Suitable carriers, excipients, etc. can be found in standard pharmaceutical texts, for example, Remington's Pharmaceutical Sciences, 18th edition, Mack Publishing Company, Easton, Pa., 1990.

The use of the strains of the invention is particularly in the form of viable cells. This means the administration of the living bacteria which allows the probiotic activity to take place in the individual gastrointestinal tract. However, as a result of the preparation process (e.g. a fermentation to form a dairy product), the culture or part of the culture may be in form of non-viable cells. As demonstrated below, the strains of the invention secrete one or more soluble factors responsible of the antibacterial activity.

In this regard, one embodiment of the present invention is a product which comprises an effective amount of the strain 7315 or of the strain 7316 or of a mixture thereof. The product could be in a suitable form for administration such as pills, capsules, microcapsules, granules, suspensions, syrups or freeze-dried powders to be administered directly. Depending on the form, the strains may be as purified bacteria, as a bacterial culture, as part of a bacterial culture, as a bacterial culture which has been post-treated, and alone or together with suitable carriers or ingredients. Prebiotics could be also added. This product may be administered to the subject as an additive.

In other embodiments, the strains of the invention may be used for the preparation of a variety of edible products, such as a milk products, a yogurt, a curd, a cheese (e.g. quark, cream, processed, soft and hard), a fermented milk, a milk powder, a milk based fermented product, an ice-cream, a fermented cereal based product, a milk based powder, a beverage, a dressing, and a pet food. Examples of other edible products are meat products (e.g. liver paste, frankfurter and salami sausages or meat spreads), chocolate spreads, fillings (e.g. truffle, cream) and frostings, chocolate, confectionery (e.g. caramel, fondants or toffee), baked goods (cakes, pastries), sauces and soups, fruit juices and coffee whiteners. However, the term "edible product" is used herein in its broadest meaning, including any type of product, in any form of presentation, which can be ingested by an animal. It covers both ready-to-eat products and products to which the product of the invention is added as a supplement or as a constituent component of the product. Particularly interesting edible products are dietary supplements and infant formulas. Dietary supplements intend to supply nutrients, (vitamins, minerals, fatty acids or amino acids) that are missing or not consumed in sufficient quantity in a person's diet (infants, pregnant women, elderly people, etc). Fodders for animal food would be also included.

In a particular embodiment of the invention, the edible product comprises a mixture of the strains 7315 and 7316 in a ratio 50:50. In such products, the strain or strains are present in an amount from about 10⁵ cfu/g to about 10¹² cfu/g, and preferably in an amount from about 10⁷ cfu/g to about 10¹² cfu/g, according to the current legislation. For the purpose of the present invention the abbreviation "cfu" shall designate a "colony forming unit" that is defined as number of bacterial cells as revealed by microbiological counts on agar plates.

The strains of the invention are produced by cultivating the bacteria in a suitable medium and under suitable conditions. The strains can be cultivated alone to form a pure culture, as a mixed culture together with other microorganisms or by cultivating bacteria of different types separately and then combining them in the desired proportions. After cultivation, the cell suspension is recovered and used as such or treated in the desired manner, for instance, by concentrating or freeze-drying.

The present invention also provides pharmaceutical compositions comprising the strains of the invention: In this regard, the pharmaceutical composition may be prepared to be administered orally in form of tablets, pills, capsules, microcapsules, granules, suspensions, syrups, freeze-dried powders, liquid preparations, etc., with the amount of bacteria to be incorporated therein being in the range from about 10⁵ cfu/g to about 10¹² cfu/g of the product, and preferably in an amount from about 10⁷ cfu/g to about 10¹² cfu/g Based upon the desired objective the person skilled in the art will select the appropriate excipients and/or carriers In a particular embodiment of the invention, the pharmaceutical composition comprises a mixture of the strains 7315 and 7316 in a ratio 50:50. Although oral administration is preferred, other forms are possible, such as injectable, rectal or topical.

In general, the compositions may comprise the strains of the invention as single probiotic agents, combinations of such probiotics or combinations with other therapeutic/nutraceutical agents depending on the condition.

The following sections describe the characterization of the strains of the invention, their specific probiotic features and their physiological effects on the gastrointestinal and immune systems. As used hereinafter, strain F35 corresponds to Lactobacillus plantarum CECT 7315 and strain F47 to L. plantarum CECT 7316.

### 1. Isolation of microorganisms

The source of microorganisms was swabs and feces from infants aged 0-5 years old. Samples were dissolved in PBS buffer pH 7.4, aliquoted and plated on different media for incubation under different conditions. Incubation time depended on the growth rate of the strain and run normally from 24 h to 7 days. Isolation of individual strains proceeded with the same selection media, then Gram staining and microscopic examination tests were performed for its initial characterization.

The selected strains F35 and F47 were those from feces samples which grew in a MRS medium supplemented with 0.05 % w/v HCl, 100 µg/l Novobiocin (Sigma), 5 µg/ml nystatin 5 µg/ml cyclohexamide (Sigma), 1 mg/l ampicilin, 10 µg/ml vancomycin and incubated at 30 °C under anaerobic conditions and pH 6.4. Gram staining showed a clear Gram+ staining, as well as bacilli morphology, non-spore-forming. Grown, isolated strains were then stored by lyophilization.

### 2. Growth parameters and biomass production

Growth efficiency, production of volatile sulfur compounds (VSC), production of Short Chain Fatty Acids (SCFA) and overall economic viability of the strains were assessed.

The selected strains were incubated in the initial selection conditions. Density of colony forming units (cfu/ml) was monitored to saturation and compared to McFarland standards (Biomerieux) using a spectrophotometer (Cecil CE 303). Samples were taken at 60 min intervals and 100 µl of culture (x2) were seeded over MRS or TSA plates at each time point. Yield, growth rate, dry weight, and nutrient consumption were also assessed at each time point. Yield was calculated according to y = (Xt - Xₒ) / (Sₜ - Sₒ)
where y is yield, Xₒ is initial concentration, Xₜ is time-point concentration, Sₒ is initial nutrient concentration and Sₜ is time-point nutrient concentration.

Growth rate was calculated according to µ = µₘₐₓ * (S/Kₛ+S)
where the saturation constant is Kₛ= 697 mg/ml and µₘₐₓ = 0.46 h⁻¹

In order to calculate dry weight, 1 ml culture was centrifuged at 10000 rpm for 5 min, discarded the supernatant, air dried at 25 °C for 72 h and weighted in a precision balance. Nutrient consumption was assessed by indirect colorimetry. Sugar content in supernatants obtained by centrifugation of culture media samples at 10000 rpm for 5 min were tested for calorie release.

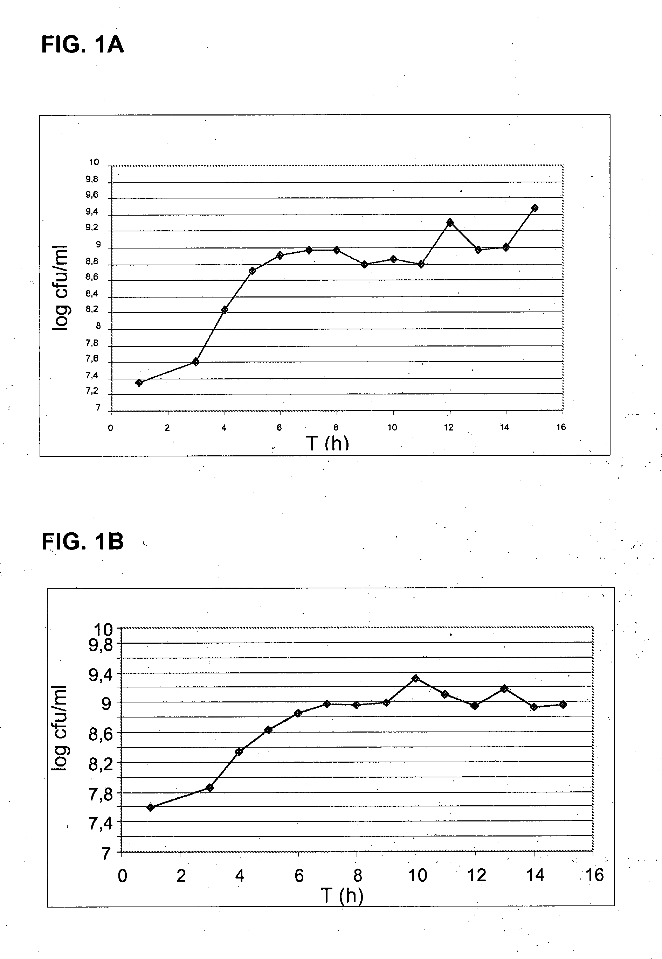

As shown in FIG. 1, the strains have a grow efficiency suitable to be industrially produced and economically viable (i.e. minimum production time and maximum yield). The µ*ₘₐₓ* was 0.4425 h⁻¹ for both strains F35 and F47.

### 3. Milk fermentation test

The aim of this test was to determine whether F35 and F47 were able of fermenting milk and whether they produced substances of unpleasant odor (VSC or SCFA). One liter of non-enriched skim milk was pasteurized and aliquots of 100 ml were inoculated with 10⁸ cfu of each individual strain F35 and F47 and incubated at 37 °C for 24 h (x3). Coagulation and odor where then estimated by eye and smelling. Results showed that both strains are capable of fermenting milk to form yogurt and none presented unpleasant odors.

pH was followed during the fermentation, resulting in a decrease until a value of pH 5.8 for F35 and 5.4 for F47 in the stationary phase. This is due to the buffer effect caused by the milk and the biomass production.

### 4. Taxonomic characterization of strains

Bacteria from selected strains were harvested, washed and pre-treated with pre-lysis buffer (480 µl EDTA 50 mM pH 8.0; 120 µl lysozyme 10 mg/ml) by resuspending and incubation at 37 °C for 60 min. DNA was extracted using Wizard genomic DNA purification kit (Promega). After centrifugation of the pre-treated bacteria at 14000 g for 2 min to remove the supernatant, the Promega's protocol was followed. In brief, bacteria were resuspended in Nuclei Lysis Solution and incubated at 80 °C for 5 min, then cooled to room temperature. Cell lysates were incubated in RNase solution at 37 °C for 60 min and proteins were precipitated by adding the Protein Precipitation Solution and vortexing at high speed. Samples were cooled down and centrifuged at 15000 g for 3 min. The supernatants containing the DNA were transferred to clean 1.5 ml microfuge tubes and mixed with 600 µl of isopropanol by inversion. DNA was collected by centrifugation at 15000 g for 2 min and carefully pouring off the supernatant. DNA samples were washed with 600 µl of 70% ethanol by gently inverting the tube several times. Ethanol was removed by aspiration, after centrifugation at 15000 g for 2 min. Finally, the DNA pellet was resuspended in 100 µl of Rehydration Solution by incubating at 65 °C for 1 h. Samples were stored at 2-8 °C.

### 4.1 Genus and specie genetic identification

The 16S rRNA was amplified by PCR using the universal primers Eub27f and Eub1492r, which produce a fragment nearly full-sequence of 16S (more than 1000 nucleotides) (TABLE 1). Then, the DNA obtained was washed using the kit Quiaquick (Quiagene).

**TABLE 1. Primers used for amplifying and sequencing the 16S gene.**

| Step | Primer | Orientation | 5' → 3' Sequence |
|---|---|---|---|
| Amplification | Eub27f | forward | GAGTTTGATCCTGGCTCAG (SEQ ID NO: 1) |
| | Eub1492r | reverse | TACGGYTACCTTGTTACGACTT (SEQ ID NO: 2) |
| Sequencing | 27f | forward | AGAGTTTGATCCTGGCTCAG (SEQ ID NO: 3) |
| | 357f | forward | CGCCCGCCGCGCCCCGCGCCCGGCCCGCC GCCCCCGCCCCCCTACGGGAGGCAGCAG (SEQ ID NO: 4) |
| | 907r | reverse | CCGTCAATTCCTTTGAGTTT (SEQ ID NO: 5) |
| | 1492r | reverse | GGTTACCTTGTTACGACTT (SEQ ID NO: 6) |

Four consecutive sequencing reactions were performed for each sample in a Genetic Analyzer 3130 (Applied Biosystems) using Big Dye kit v.3.1, using the primers shown in TABLE 1. Data collection and chromatograms were built using DNA Sequence Analysis v.5.2 software (Applied Biosystems) and checked by visual analysis with Chromas (Technelysium Pty Ltd.) and BioEdit (Ibis Biosciences).

Genus Identification was carried out using the Ribosomal Database Project (RDP) (Q. Wang et al., "Naive Bayesian Classifier for Rapid Assignment of rRNA Sequences into the New Bacterial Taxonomy", Appl. Environ. Microbiol. 2007, vol. 73, pp. 5261-7) tool. Species identification was performed by comparison of the obtained sequence with 16S sequences of known organisms from both RefSeq data base (http://www.ncbi.nlm.nih.gov/RefSeq/) by means of a BLASTN, and from Ribosomal Database Project (http://rdp.cme.msu.edu/, J.R. Cole et al, "The Ribosomal Database Project (RDP-II): introducing myRDP space and quality controlled public data" Nucl. Acids Res. 2007, vol. 35, pp. 169-72).

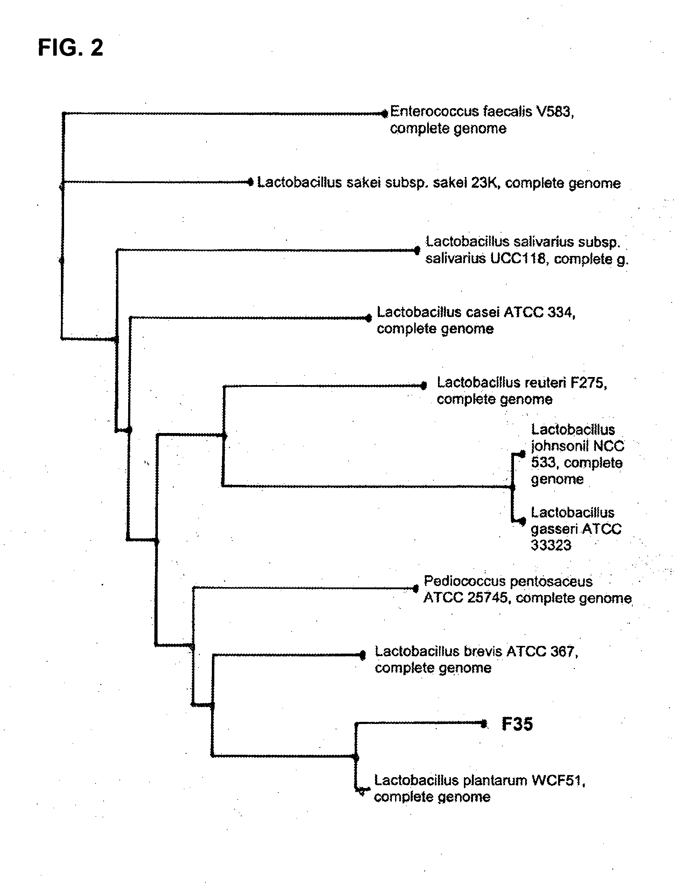

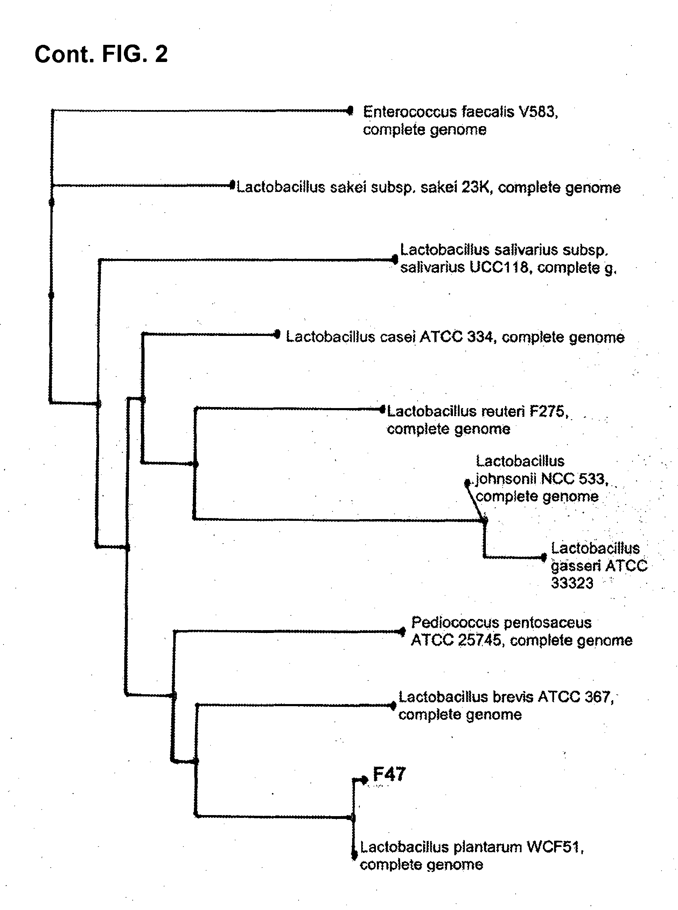

RDP tool identified both strains F35 and F47 as belonging to the Lactobacillus genus. Results showed that F35 and F47 present 98% and 99% identity with 16S from L. plantarum strain WCFS1 respectively (FIG. 2).

### 4.2. Strain genotyping

Characterization was performed by genomic digestion and pulsed-field gel electrophoresis. F35 and F47 strains were subjected to a previously described protocol (A.M. Rodas et al., "Polyphasic study of wine Lactobacillus strains: taxonomic implications", Int J Syst Evol Microbiol 2005, vol. 55, pp. 197-207). Strains were grown on MRS agar plates and incubated at 37 °C 5% CO₂ for 18 h. Then harvested and washed 3 times in 8 ml PET (10 mM Tris pH 7.6, 1 M NaCl) then centrifuged at 6000 rpm 10 min. Pellets were resuspended in 700 µl lysis buffer (6 mM Tris, 1 M NaCl, 0.1 M EDTA, 0.5% SLS, 0.2 % deoxycholic acid; 1 mg/ml lysozyme; 40 U/ml mutanolysin; 20 µg/ml RNase). An equal volume of 1.6% low melting point agarose (FMC BioProducts, Rockland, ME, USA) was added to the resuspended cells and solidification was allowed at 4 °C for 1 h. Inserts were transferred to 2 ml lysis buffer II (0.5 M EDTA pH 9.2, 1 % N-lauryl sarcosine and 1 mg/ml pronase) and incubated at 50 °C for 48 h. Then inserts were washed at room temperature with TE buffer (10 mM Tris, 1 mM EDTA pH 8.0). Total DNA digestion was performed by Sfi-I and Sma-I restriction enzymes (Roche Diagnostics).

Pulse-field electrophoresis was carried out using CHEF DRIII apparatus (BioRad Laboratories). Inserts were loaded in a 1% agarose gel (SeaKem ME agarose, FMC BioProducts, ME, USA). TABLE 2 describes electrophoresis conditions for each enzyme. DNA MW markers were Lambda ladder PFG Marker and Low Range PFG Marker (New England Biolabs). After electrophoresis, gels were stained with ethidium bromide and UV using GelDoc System (BioRad).

**TABLE 2. Electrophoresis conditions for this study.**

| Enzyme | Block | Initial Pulse (sec) | Final Pulse (sec) | Time (hours) |
|---|---|---|---|---|
| Sfi-I | 1 | 2 | 10 | 10 |
| | 2 | 15 | 25 | 6 |
| Sma-I | 1 | 0.5 | 5 | 16 |

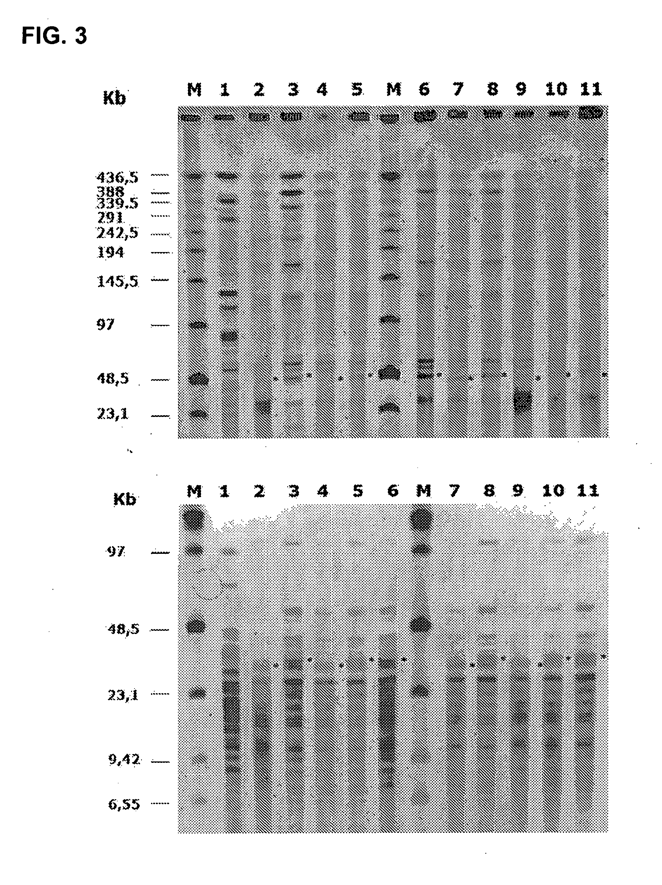

The results obtained with both enzymes Sfi-I and Sma-I were similar. As is shown in FIG. 3, the pattern obtained for the strain L. plantarum 299v is very different from the patterns shown by F35 and F47 strains. These patterns differ in the position of a band in case of Sfi-I (upper figure), while with Sma-I enzyme (lower figure) strain F47 shows an additional band. In literature, it has been described that unlike other Lactobacillus, L. plantarum specie presents a high genetic heterogeneity (I. Sánchez et al., "Polyphasic study of the genetic diversity of lactobacilli associated with 'Almagro' eggplants spontaneous fermentation, based on combined numerical analysis of randomly amplified polymorphic DNA and pulsed-field gel electrophoresis patterns" Journal of Applied Microbiology 2004, vol. 97, pp. 446-58). Strains F35 and F47 look genetically very close related, so they may come from the same clone origin (F.C. Tenover et ol., "Interpreting chromosomal DNA restriction patterns produced by pulsed- field gel electrophoresis: criteria for bacterial strain typing" J. Clin. Microbiol. 1995, vol. 33, pp. 2233-9).

### 5. Resistance to gastrointestinal environment

In order to evaluate the resistance of F35 and F47 strains to the conditions of the gastrointestinal environment of mammals, specific assays were performed for quantifying survival after treatment with lysozyme, acidic environment, bile salts and oxygen peroxide.

5.1 Tolerance to lysozyme: 20 µl aliquots of each bacterial strain culture were placed in a 96-well plate, and then 200 µl lysozyme (Sigma) at 100, 200 or 300 µg/ml in MRS medium were added. Plates were incubated at 37 °C, 5% CO₂ for 1 h. Bacterial growth was quantified by measuring optical density at 620 nm in an ELISA reader. Results are expressed vs the control, which is the maximum growth of each of the strains in a MRS broth (TABLE 3).

**TABLE 3**

| Lysozyme (µg/ml) | Mean | Mean C+ | Standard deviation | Standard deviation C+ |
|---|---|---|---|---|
| F35 | | | | |
| 100 | 0.35 | 0.25 | 0.02 | 0.02 |
| 200 | 0.32 | 0.24 | 0.01 | 0.01 |
| 300 | 0.38 | 0.28 | 0.01 | 0.02 |

| F47 | | | | |
|---|---|---|---|---|
| 100 | 0.16 | 0.20 | 0.01 | 0.01 |
| 200 | 0.18 | 0.21 | 0.01 | 0.01 |
| 300 | 0.19 | 0.20 | 0.02 | 0.02 |

5.2 Tolerance to acidic environment: 20 µl aliquots of each strain culture were placed in a 96-well plate, then 200 µl aliquots of TBS or MRS media adjusted to desired pH value with HCl (Panreac) were placed over bacteria containing wells. Plates were then kept at 42 °C for 2 h and read in a spectrophotometer at 620 nm. Results are expressed vs the control, which is the maximum growth of each of the strains in a MRS broth (TABLE 4).

**TABLE 4**

| PH | Mean | Mean C+ | Standard deviation | Standard deviation C+ |
|---|---|---|---|---|
| F35 | | | | |
| 2 | 0.1976 | 0.428 | 0.02 | 0.018 |
| 2.5 | 0.2547 | 0.277 | 0.022 | 0.031 |
| 3 | 0.2498 | 0.366 | 0.019 | 0.018 |
| 3.5 | 0.2494 | 0.472 | 0.017 | 0.015 |
| 4 | 0.263 | 0.404 | 0.019 | 0.017 |
| 4.5 | 0.285 | 0.345 | 0.02 | 0.025 |
| 5 | 0.2783 | 0.266 | 0.0278 | 0.026 |
| 5.5 | 0.18 | 0.173 | 0.017 | 0.021 |
| 6 | 0.3465 | 0.355 | 0.0201 | 0.018 |

| F47 | | | | |
|---|---|---|---|---|
| 2 | 0.1392 | 0.2324 | 0.019 | 0.017 |
| 2.5 | 0.2748 | 0.3506 | 0.025 | 0.024 |
| 3 | 0.205 | 0.27 | 0.017 | 0.016 |
| 3.5 | 0.157 | 0.207 | 0.026 | 0.024 |
| 4 | 0.1963 | 02406 | 0.011 | 0.015 |
| 4.5 | 0.2477 | 0.286 | 0.09 | 0.05 |
| 5 | 0.31 | 0.373 | 0.017 | 0.016 |
| 5.5 | 0.308 | 0.337 | 0.015 | 0.014 |
| 6 | 0.2929 | 0.282 | 0.017 | 0.014 |

5.3 Tolerance to bile salts: 20 µl aliquots of each bacterial strain culture were placed in a 96-well plate. 200 µl bile salts dilution were added to wells. Four conditions and three different concentrations (0.3%; 0.5% and 1%, in w/v, pH 3 and 0.3% w/v pH not adjusted) were assayed. Plates were kept at 37 °C, 5% CO₂ for 3 h and read at 620 nm (TABLE 5).

**TABLE 5**

| Bile salts-pH | Mean | Mean C+ | Standard deviation | Standard deviation C+ |
|---|---|---|---|---|
| F35 | | | | |
| 0.3% no pH | 0.217 | 0.4001 | 0.017 | 0.015 |
| 0.3% pH 2 | 0.2214 | 0.4009 | 0.018 | 0.017 |
| 0.5% pH 3 | 0.3154 | 0.4099 | 0.02 | 0.019 |
| 1%pH3 | 0.2997 | 0.4099 | 0.022 | 0.025 |

| F47 | | | | |
|---|---|---|---|---|
| 0.3% no pH | 0.525 | 0.41 | 0.02 | 0.017 |
| 0.3% pH 2 | 0.7728 | 0.419 | 0.017 | 0.022 |
| 0.5% pH 3 | 0.7224 | 0.42 | 0.019 | 0.025 |
| 1%pH3 | 0.756 | 0.4194 | 0.022 | 0.026 |

5.4 Tolerance to oxygen peroxide: 20 µl aliquots of each strain culture were placed in a 96-well plate. 200 µl H₂O₂ dilutions (0.8823, 0.5882, 0.2941 mM ) were added to wells and plates incubated 30 min at 37 ° C and then read at 620 nm (TABLE 6).

**TABLE 6**

| H₂O₂ dilution (mM) | Mean | Mean C+ | Standard deviation | Standard deviation C+ |
|---|---|---|---|---|
| F35 | | | | |
| 0.8823 | 0.32 | 0.3021 | 0.016 | 0.014 |
| 0.5882 | 0.332 | 0.3024 | 0.018 | 0.018 |
| 0.2941 | 0.311 | 0.301 | 0.016 | 0.015 |

| F47 | | | | |
|---|---|---|---|---|
| 0.8823 | 0.272 | 0.31 | 0.022 | 0.02 |
| 0.5882 | 0.3424 | 0.32 | 0.016 | 0.018 |
| 0.2941 | 0.286 | 0.33 | 0.021 | 0.023 |

A result of or more than 40% of growth in respect of the control, is considered as positive. Considering agents in the mouth which have a bactericide activity (oxygen peroxide and lysozyme), no lack of viability of the strains was observed, even in concentrations of the bactericide agents greater than the physiological conditions (0.8823 mM/ml lysozyme and 10 µg/ml oxygen peroxide). Regarding the parameters in the stomach (pH), there was only a lack of viability of 5% at pH 2 (very strict conditions, not physiological). There was no lack of viability in the test with bile salts, even in the moment of the stomach emptying when the detergent capacity of the bile salts is combined with an acid pH.

### 6. Adhesion to intestine

Mucus was obtained by washing an intestine with PBS pH 7.4 gelatin 0.01% and proteases inhibitor (Complete^{®}, Sigma). The mucosa was scrapped and deposited in a recipient with buffer 10 mM HEPES-Hank's salt pH 7.4 and the same inhibitors. Mucus was then washed by centrifugation at 13000 rpm for 10 min with the same buffer. The supernatants were recovered and mucus content was evaluated by Bradford protocol.

The adhesion capability of F35 and F47 were compared to the adhesion of two commercial strains. Inoculums of 150 µl of overnight cultures of axenic cultures of each of the microorganisms were put in MRS medium supplemented with tritium-labeled thymidine (5 µl in 3 ml of MRS) and microorganisms concentration counted in a Neubauer chamber. The preparations were then centrifuged and pellets resuspended in PBS to obtain the desired cfu/ml. The tritium signal incorporated to the microorganisms is calculated from the initial tritium signal (the µl of tritium-labeled thymine added to the medium) and the supernatant signal. From the total cfus from the growth, the ratio between this number (signal incorporated to the biomass) and the total number of microorganisms in the growth results in dpm/cfu (signal/bacterium).

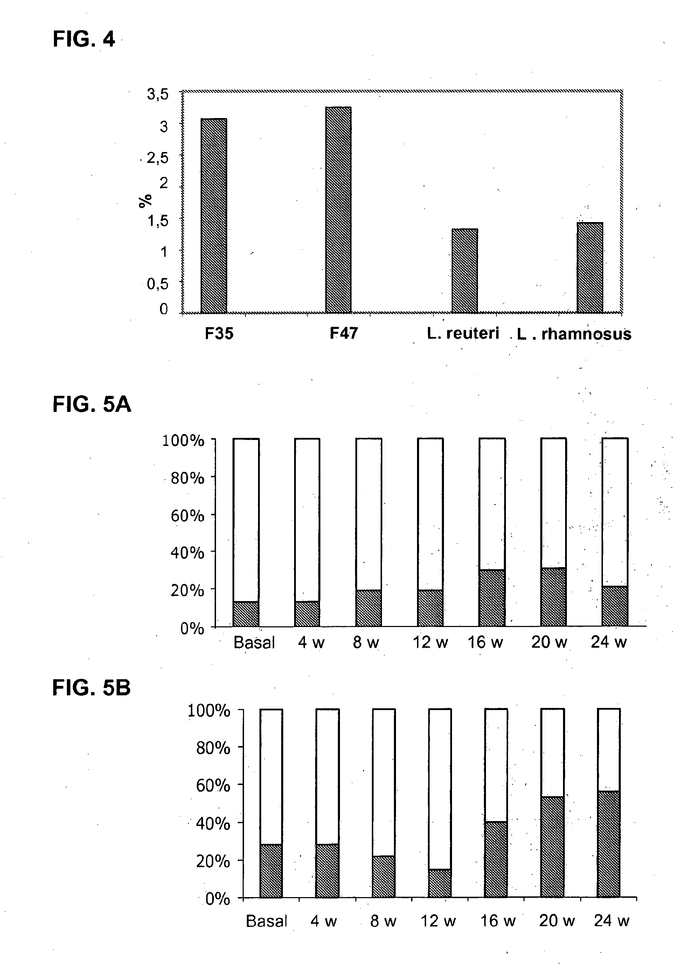

To carry out the assay, it was considered that the mucus does not have unlimited points for adhesion of microorganisms and that in a limited area such as a plate well, the addition of an inadequate concentration of cfus may result in a super or undervaluation of the binding capability. Thus, in a pre-assay, the % of adhesion of different concentrations was evaluated to find the adequate concentration to be used in the assay. A decrease in the % of adhesion was observed from a concentration of 1x10⁸ cfu/ml, so this concentration was chosen for the assay. 24 h before the assay, 1 ml of the mucus solution 0.5 mg/ml was incubated in wells of ELISA plate. Then, the microorganisms preparations were added to the wells and after incubation for 60 min at 37 °C, the supernatants of each well were removed, wells washed with MEM Alpha medium (Gibco) and scrapped to unstick the mucus-microorganisms from the wells. The % of adhesion is calculated by counting tritium signal in a scintillation reader of the mucus preparation in the well and the dpm/cfu as obtained above. The result is the number of bacteria adhered per unit of mucus area. FIG. 4 shows that 3.06 x 10⁶ and 3.25 x 10⁶ cfu of the strains F35 and F47 respectively can bind to 2 cm² of intestinal mucus. Comparing with other commercial strains, F35 and F47 showed double adhesion capability.

### 7. Toxicity assays

Strains F35 and F47 were administered at 5 x 10¹⁰ cfu/kg in PBS to eighteen 9-weeks old Wistar rats (male and female) over two consecutive days to a total dose of 10¹¹ cfu/kg. Animals were fed with fodder (Teklacd 2014) and water ad libitum. Administration was after eating over a full stomach and aided by an oro-gastric tube. Every two days, animal behavior and wellbeing was determined by assigning values to parameters as detailed in TABLE 7. Total score results from the sum of the values obtained in each parameter: weight + behavior + response to stimuli. No negative effects on animal behavior and well-being were noted during the study.

**TABLE 7. Evaluation of animal behavior and wellbeing. 0 value is normal; a value of 7 indicates intolerable morbidity and animal sacrifice to avoid unnecessary suffering.**

| | Parameter | value |
|---|---|---|
| Weight | Normal | 0 |
| | Loss < 10% | 1 |
| | Loss 10%-20%; possible change in feces | 2 |
| | Loss > 20% | 3 |
| Behavior | Normal | 0 |
| | Fur damaged | 1 |
| | Fur damaged and ocular secretion | 2 |
| | Abnormal posture | 3 |
| | Self mutilation or painful utterance | 3 |
| Response to stimuli | Normal | 0 |
| | Aggressive | 3 |
| | Comatose | 3 |

Animals were sacrificed on day 7 by CO₂ inhalation. A full necropsy was performed in order to find macroscopic organ damage. Samples of mesenteric lymph nodes were taken to assay for bacterial translocation. Approximately 5 mg of each sample were homogenized in 1 ml 0.01 % gelatin PBS. 100 µl-from this homogenate were plated either on McConkey plates or MRS plates. Colonies were counted after incubation at 37 °C for 48 h. Results are displayed in TABLE 8. There were a few lactic colonies observed in MRS plates from control, F35, or F47-fed animals. These correspond to a normal basal translocation of lactic acid bacteria (J.S. Zhou et al, "Acute oral toxicity and bacterial translocation studies on potentially probiotic strains of lactic acid bacteria", Food Chem Toxicol 2000, vol. 38, pp. 153-61).

**TABLE 8. Bacterial translocation to the mesenteric lymph nodes of fed animals. Numbers indicate the number of animals with positive bacterial growth and the maximal number of cfus per milligram of tissue.**

| Group | Sex | Enterobacteria [cfu/mg] | Lactic acid Bacteria [cfu/mg] | Maximum observed Translocation [cfu/mg] |
|---|---|---|---|---|
| Control | Males | 0/3 | 2/3 | 2 |
| | Female | 0/3 | 2/3 | 2 |
| F35 | Males | 0/3 | 1/3 | 2 |
| | Female | 0/3 | 2/3 | 4 |
| F47 | Males | 0/3 | 1/3 | 4 |
| | Female | 0/3 | 0/3 | 0 |

In conclusion, the results show that oral administration of strains F35 and F47 is safe since they do hot lead to an increase in translocation of lactic bacteria nor do they facilitate enterobacterial translocation. All the animals showed a similar body weight evolution along the study. No significant differences were observed in the fodder and water consumption. Neither clinical symptom nor alteration of the animals' wellbeing was detected. No macroscopic damages in organs and cavities were detected during the histopathological examination.

### 8. Anti-bacterial properties

In order to asses whether strains F35 and F47 presented ant-bacterial activities, a Campbell protocol was performed using agar plates seeded with bacterial pathogens in Oxoid medium. Pathogens used in this study were selected among those commonly present in the human gastrointestinal tract (see TABLE 9). Plates were swabbed uniformly and grown to confluence at the appropriate temperatures in a 5% CO₂ incubator. Then, a cylinder section of an uniformly seeded, confluent F35 or F47 agar plate was placed loan-to-loan over the pathogen plate and incubated overnight at 37 °C.

Next day, inhibition zones were measured by placing the agar plate over a flat rule. Growth inhibitory activity was then calculated by subtracting the cylinder diameter (CD) from the inhibition zone diameter (IZD) and dividing this difference by two following the formula GI = (IZD-CD) / 2

**TABLE 9. Growth inhibition values according to the formula depicted above.**

| | F35 | F47 |
|---|---|---|
| Salmonella enterica Enteritidis CECT 4155 | 1 | 1 |
| Salmonella enterica Typhimurium CECT 4594 | 0.5 | 1 |
| Bacillus subtilis CECT 35 | 1.5 | 0.5 |
| Clostridium botulinum CECT 4611 | 1 | 0.5 |
| Yersinia enterocolitic NCTC 10460 | 2 | 2.5 |
| Listeria monocytogenes CECT 4031 T | 0.5 | 0 |

F35 and F47 strains inhibited growth of the pathogenic strains shown in TABLE 9. Thus, they benefit the intestinal microbiota balance due to their capacity to inhibit the growth of pathogenic strains.

### 9. Secretion assay for antibacterial agents

In order to test whether an antibacterial factor from F35 and F47 was secreted to the media, a 1:1 mixture of both strains was plated on MRS plates and incubated 18 h at 37 °C and 5% CO2. Then bacteria were harvested and pelleted at 6000 rpm for 15 min. Supernatant was filtered through a 0.22 µm filter (Millipore) and pellet was washed twice and resuspended in HEPES (Sigma). McConkey plates were seeded with each pathogenic strain (Salmonella enterica CETC 4155, Salmonella typhimurium CETC 4594 or Escherichia coli entero-haemorragica (H7:O157, wild isolate)) and cylindrical 5 mm diameter wells were pierced on the plates with a pasteur pipette. Wells were then filled with 60 µl of each resuspended mixture of strains or supernatant and plates were incubated at 37 °C for 24 h. Next day the diameter of inhibition zones was calculated as, above. The results, shown below in TABLE 10, indicate that growth inhibition is due to one or more soluble factors secreted by these two strains.

**TABLE 10. Growth inhibition values for supernatant vs. cells of strains F35 and F47**

| Fraction | S. enterica CECT 4155 | S. enterica CECT 4594 | E. coli EHEC |
|---|---|---|---|
| Whole cells | 0.0 ± 0.0 | 0.0 ± 0.0 | 0.0 ± 0.0 |
| Supernatant | 2.0 ± 0.0 | 1.6 ± 0.5 | 1.4 ± 0.2 |

### 10. Immunomodulation in humans

The aim of this clinical assay was to describe the immunomodulatory effects of strains F35 and F47 in humans. The study was randomized, doubly-blinded and controlled with placebo. Sixty healthy individuals aged 65-70 year old chosen under a strict exclusion criteria consisting on: any serious acute disease within one month previous to assay; any advanced neoplasic disease; intolerance to dairy products; swallowing disorders which impede the intake of the preparation under study; diet supplementation (vitamins/oligoelements) within one month previous to assay; not able to attend to monitoring appointments. L. plantarum strains F35 and F45 were mixed 1:1 before dosage. There were three groups:
Low dose (n=20): 5 x 10⁸ cfu/day in 20 g powdered skim milk.
High dose (n=20): 5 x 10⁹ cfu/day in 20 g powdered skim milk
Placebo (n=20): 20 g powdered skim milk (placebo/vehicle)

Individuals received one of three treatments randomly over 12 weeks and they were monitored during 12 further weeks. Each dose was packed in a vacuum sealed envelope to be dissolved in 200 ml water or other cold drink. In the monitoring appointments, the following data, among other, were collected: physical examination; clinical history (chronic diseases, surgery, defecations/week); self-medication habits; height, weight, BMI; life quality questionnaire (SF-36); routine blood analytics (hemogram, leukocyte count, coagulation test; glycemia, cholesterol, triglycerides, urea, creatinine, ionogram, bilirubin, C-reactive protein) and immunology tests.

### Immunology tests:

The following data were collected: leukocyte populations (total T cells (CD3+), T cells CD4+, T cells CD8+, activated T cells (CD25+), B cells (CD19+), NK cells (CD56+), and APCs (HLA-DR+)) and cytokines: IL-1, IL-10 and TGF-β.

Antibodies: PECy5 conjugated mouse anti-human CD3, PE conjugated mouse anti-human CD25, APC conjugated mouse anti-human CD8, FITC conjugated mouse anti-human CD4, FITC conjugated mouse anti-human CD3, APC conjugated mouse anti-human CD19, PE conjugated anti-human CD56/16, PECy5 conjugated mouse anti-human HLA-DR

Reagents and kits: (x10) FACSlysing Buffer Solution and BD™ CBA Flex Sets: human IL-1β and human IL-10 provided by BD Bioscience (San Diego, CA, USA); ELISA for human TGF-β1 provided by Bionova cientifica, S.L. (Madrid, Spain). All the experiments were made at room temperature.

Phenotypic characterization by flow cytometry: After titering the labeled antibodies with fluorochromes by cytometry, 2 tubes with 50 µl of total blood each one, were simultaneously processed: tube 1 with 2 µl of anti-human CD25PE, 4 µl anti-human CD4 FITC, 5 µl anti-human CD8 APC and 2 µl anti-human CD3 PECy5, and tube 2 with 2 µl anti-human CD56/16 PE, 2 µl anti-human CD3 FITC and 2 µl anti-human CD19 APC. After incubation for 15 min at darkness; 2 ml FACSlysing Buffer Solution was added, 7 min at darkness to cause erythrocytes lysis. Then, it was centrifuged (1500 rpm, 10 min) and pellets washed twice with PBS (1500 rpm, 10min). The pellet was resuspended with PBS and analyzed by FACScalibur. Values obtained by cytometry express the percentage of each phenotype marker at 10000 cells CD3+.

Analysis of plasmatic IL-10 and IL-1β by flow cytometry: It was analyzed with the Kit Human Soluble Protein Master Buffer (BD Cytometric Bead Array) following manufacturer instructions. In brief, 50 µl of plasma obtained by routine procedures were incubated 1 h with 50 µl of a mix containing beads coated with anti-IL-10 antibodies and beads coated with anti-IL-1β antibodies. Then, 50 µl of a solution containing specific antibodies PE-labeled against other cytokines epitopes were added for 2 h. Then, the sample was washed and resuspended for analysis by FACScalibur. Cytometric analysis of the cytokines was based on detection of PE combined with the different size of each bead. Results were normalized with a standard curve from IL-10 and IL-1β provided by the manufacturer (pg/ml).

Analysis of TGF-β1 by ELISA: A sandwich type ELISA was used following manufacturer instructions. In brief, plasma coming from blood samples were diluted 1:50 to then acidified with HCl 1N and neutralized with NaOH 1 N (1 h). With this procedure, the TFG-β1 was activated. Dilutions were prepared from 600 pg/ml of TGF-β1 in order to draw a standard curve. Then, 100 µl of standards and of each sample were deposited x2 in a 96-well plate and incubated overnight at 4°C. Then, 100 µl of mouse anti-TGF-β1 monoclonal was incubated 2 h in a shaker, and next, 100 µl of biotin-labeled mouse IgG antibody was incubated for 45 min with shaking. After consecutive washings 100 µl of a solution of streptavidine peroxidase was dispensed to each well and incubated 45 min with shaking. Next, 3 washings were done and 100 µl of solution with peroxidase substrate (TMB). Fifteen minutes later the solution stop was added and the absorbance (450 nm) measurement was carried out. The values of concentration of the samples were obtained by extrapolation of absorbance to the lineal plot of the standard curve.

Statistical study was made with SPSS v12 software (USA). Intragroup comparisons were made with the Wilcoxon signed-rank test and intergroup comparisons with the Kruskal-Wallis test.

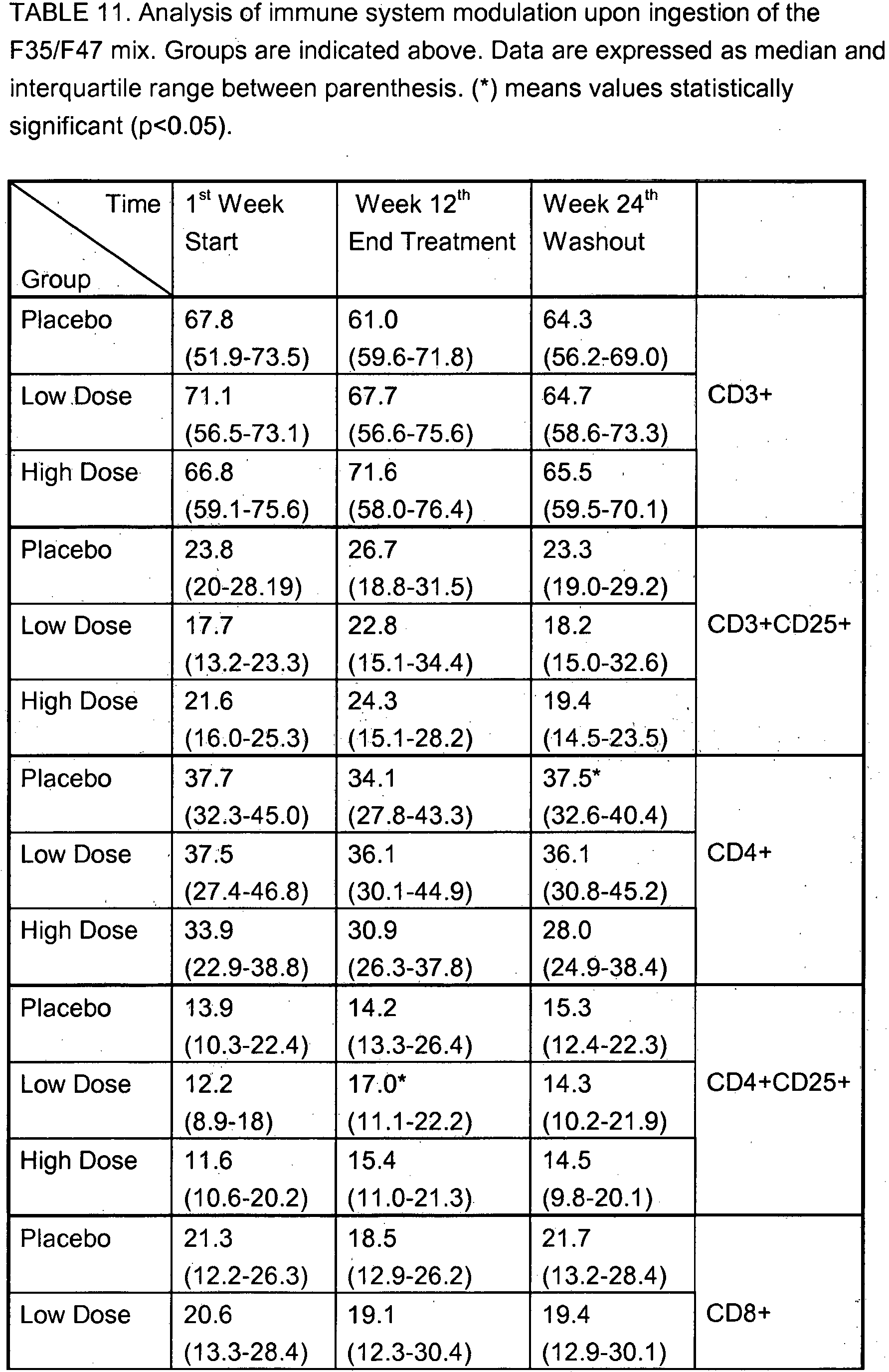

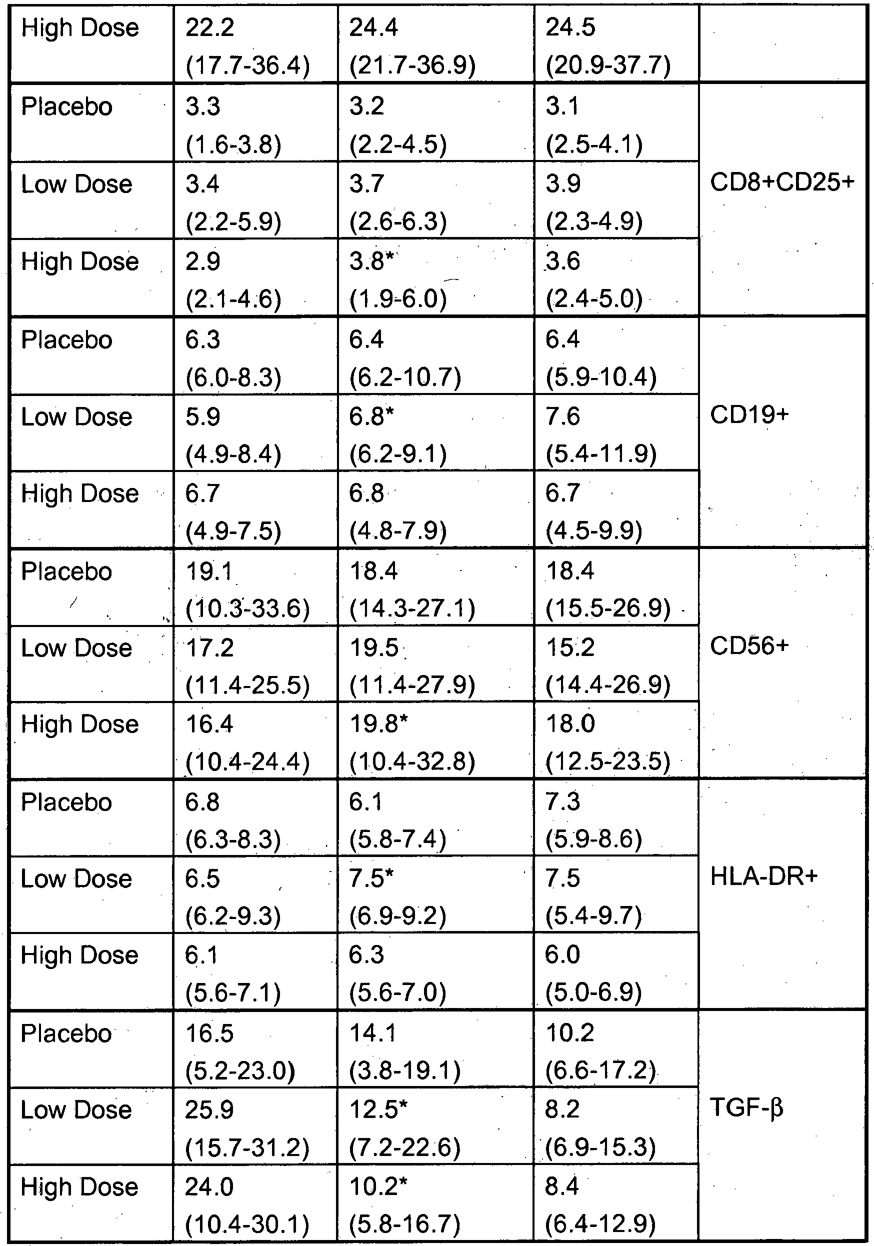

According to these data, treatment with a mix of strains F35 and F47 results in an overall activation of the immune system. Particularly, CD4+ cells and CD8+ cells showed a significant increase in activity as indicated by CD25 expression. B lymphocytes (CD19+) and Natural Killer cells (CD56+) showed a significant increase in number and antigen presenting cells (HLA-DR+) were stimulated as well. Further, a significant decrease in pro-inflammatory cytokine release (TGF-β) was observed. This result, together with the cellular immunity retrieval, may indicate that the immune regulation moves from a more unspecific level (cytoquines) towards a more specific level (cellular). The levels of cytokines IL-1 and IL-10 were undetectable in all the groups since the subjects under study were healthy.

Defecations/week was monitored during the study clinical to evaluate the regulation of the intestinal transit by the administration of the preparation. The results (FIG. 5) show that subjects supplemented with the preparation have an enhanced frequency in their defecating habits, which indicates thet the strains of the invention have a regulating effect of the intestinal transit.

Taken together, these data demonstrate that the systemic immunity is regulated due to the intestinal interaction with a mixture of Lactobacillus plantarum strains (F35 and F47), stopping the inflammatory immune signalling pathway.

### 11. In vitro evaluation of the immunomodulation ability of cytokine production of a probiotic in an intestinal mucosa model.

As will be exposed below, the inventors surprisingly found a group of Lactobacillus plantarum strains that were able to antagonize the inflammatory capacity of some pathogenic bacteria usually found in the intestines. These strains object of the invention share an improved immunomodulation activity in respect of other related bacterial strains. The immunomodulation is translated into a common improved induction of cytokine pattern from the intestinal mucosa cells. The two strains F35 and F47 promote a reduction of the TNF-α and an increase of the IL-10 levels. This effect is performed using the strains in a viable form, which is the form to be applied when administered as probiotics in humans. In the sense of the invention "viable form" means that the bacteria are used alive without any previous treatment. Moreover, this common modulation of the pattern of cytokines is better than the modulation observed by other Lactobacillus plantarum assayed in non-viable forms. Members of intestinal commensal microflora are in permanent contact with GALT (Gut Associated Lymphoid Tissue). Depending on the composition of the microflora, there is a pattern of cytokine production by the immune system. This production of cytokines is developed by the Toll Like Receptors (TLR) of the dendrytic cells, when they recognize specific surface structures of the bacteria. Concretely, the most important TLR receptors in this function are TLR-2 and TLR-4. These receptors recognize molecules, structures or highly conserved residues along the different microbial taxa, that are expressed on the surface of the same and act as ligands to TLR . When TLR-2 and 4 interact with certain bacterial antigens, they start the production of inflammation markers. In case, that as a consequence of the microbiota composition, the production of cytokines becomes excessive, it is possible that injuries in the tissues could appear as a result of inflammation and immunologic impairments. These injuries attain primary consequences, including a lesser efficiency of the immune system response and a greater tendency to develop autoimmune diseases.

It is known that the Gram negative bacteria show on its cell surface the molecule LPS (lipopolysaccharide). LPS is an endotoxin recognized by the immune system as a potential hazard. When LPS interacts with the TLR-2 and TLR-4, it induces the production of inflammatory markers. In a normal commensal microflora composition, we can find Gram negative microorganisms; consequently, there exist contact between immune system associated to the gastrointestinal tract and the lipopolysaccharide (LPS).

Probiotic supplementation can change this situation to favour one greater presence of gram positive bacteria (grouped in the lactic acid bacteria group), with better ecologic fitness or with antagonistic properties against some Gram negative microorganisms, thus reducing the presence of LPS in the intestinal mucosa. Nevertheless, the molecular and structural configuration of the cell surface of some probiotic microorganisms, shows the ability to modulate per se the production of communication molecules between the cells of the immune system (cytokines) displacing to a most balanced pattern between pro/anti-inflammatory signalling, (without reducing the number of Gram negative bacteria).

The TLR-2 and 4 receptors are commonly expressed in the intestinal mucosa in vivo. Nevertheless, the most of the cell lines used as intestinal mucosa model, as HT-29 or Caco-2, do not have the ability to produce cytokines with or without the presence of induction elements. For this reason, inventors selected a proper cell line in order to develop the in vitro assays as shown below.

The selected model is the monocyte cell line THP-1, due to its sensitivity to bacterial components like LPS (as inductor of the inflammatory response), and its susceptibility to modulate its cytokine production when there are molecules in the medium suitable for the induction of the production of an anti-inflammatory cytokine pattern.

It was hypothesised that a probiotic (mixture of F35 and F47, by now on named Lp 3547), would have the ability to regulate the production of TNF-α (inflammatory response), in a THP-1 culture induced with LPS, showing better results that Lactobacillus plantarum 299V (named 299V for the purposes of the invention). In fact, the inventors wanted to assess if the effect of the two strains together, would be stronger than the effect of each separate strain and than the effect of prior art strains. In the following experiments with probiotic Lp 3547 and THP-1 cells, it is used LPS as inductor. One reason to choose LPS is the high reactivity that cells show against this molecule, so it provides an easier way to make the experimental readings. The other main reason is that LPS represents a realistic element of intestinal microbiota, because as indicated above in the intestine we can find Gram negative bacteria (with LPS).

The election of Lactobacillus plantarum 299V as the control of the experiment was due to its notoriety as probiotic in the Lactobacillus plantarum taxonomic group, the same where the strains F35 and F47 belong, as well as the probiotic Lp 3547 to be tested.

The set of experiments were designed with the aim to obtain information useful, for the next hypothesis:
a) probiotic Lp 3547 has a greater immunomodulation ability than Lactobacillus plantarum 299V, in relation to the regulation of cytokine profile expression (ratio of anti-inflammatory and pro-inflammatory cytokines); and
b) the effect of the strains integrated in the probiotic Lp 3547 (F35 and F47) show a greater effect than the effect by both strains separately.

The first step of the experiment was the growth of THP-1 cells obtained from the ATCC (American Type Culture Collection). They were grown in DMEM medium, until the optimum moment for the accomplishment of the immunomodulation study. The cells were cultured in 24-wells ELISA plates to a final concentration of 106 monocytes/well, approximately.

These cells, at the beginning of the experiment, are stimulated with diluted LPS in 500 microliters of DMEM medium to a final concentration of 10 ng/ml, during 2.5 hours. After this time, wells are washed three times with 0.5ml of PBS. The next step consists on putting the cells THP-1 in contact with probiotic microorganisms. The probiotic strains are previously grown over night (F35, F47 and L299V) in medium MRS at 37 °C with a 5 % of CO2 atmosphere. After the incubation, it is valued the concentration of microorganisms/ml using the Neubauer-counting chamber, in order to calculate the dilution necessary to obtain a ratio cfu/monocytes of 25:1 (2.5 x 107), in 500 microliters of DMEM, in the ELISA-wells with THP-1 cells. Each dilution of F35, F47 y L299V, is generated with DMEM medium supplemented with gentamicin (50 micrograms/ml), ampicillin (10 micrograms/ml) and chloranfenicol (12 micrograms/ml) (G.A.C abbreviated). The argument to use these antibiotics is to avoid the proliferation of microorganisms that could disturb the homogeneity of 25:1 ratio. Those antibiotics were established as innocuous for THP-1 cells and only bacteriostatics for the strains, L. plantarum F35, F47 y L. plantarum 299V. The time of co-incubation of THP-1 cells with probiotics is 24 hours, taking samples of each co-incubation at 6 hours and at the end of the experiment. The estimated most suitable times to take the samples of supernatant and to value the production of TNF-α and IL-10, are at 6 and 24 hours after the addition of the probiotic strains. The election is not random, but is product of a previous work that studied the times when the differences and values of concentration of the interleukins are more evident (data not showed). The supernatant so obtained is used to detect TNF-α and IL-10 with a commercial kit that requires an analysis by flow cytometry, as that used in section 10 before, and performing the routinely steps well known by the skilled person.

For the interpretation of the obtained results, it was calculated the slope between the obtained values at 6 and 24 hours, calculating later the normalized slope. Normalized slope is calculated with the following formula: NS= ((1 - IL value 24h / IL value 6h) / 24) x 100;
wherein NS is the normalized slope, and IL value is the concentration of the interleukin 10 or TNF-α at 6 or 24 hours. The reason for selecting this method is to obtain a standard value that allows a transversal comparison between the experiments (Table 13), since it is more interesting the evolution of cytokines concentration than the absolute value of them (pg/ml). In the values obtained at 6 hours, THP-1 cells are still under induction of LPS; then, the concentration of TNF-α is elevated and the IL-10 reduced. It is at 24 hours when it is possible to observe the reversion of the cytokine production profile (reduction of TNF-α and the increase of IL-10).

In TABLE 12 the experimental design of each test is schematically shown. The tests, Exp.1, Exp.2, Exp.3, Exp.4 and Exp.5, have the purpose to attribute the cytokines detection to its production by THP-1 cells, and to fix that the values obtained are not a result of crossed reactions with other integral elements of the experiment. The Exp.6 represents the basal values of cytokines production without inductor neither probiotics (negative control). Exp.7 represents the production value of cytokines by THP-1 when induced by LPS (positive control). The rest of tests (Exp.8 - Exp. 13) correspond to the valuation of cytokine profile's modulation by the tested probiotic strains. All the values shown are the averages of duplicates in each replica realized.

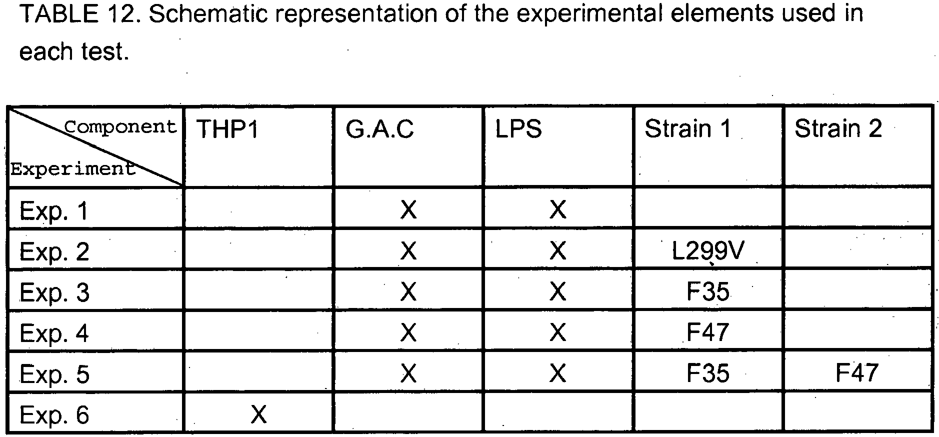

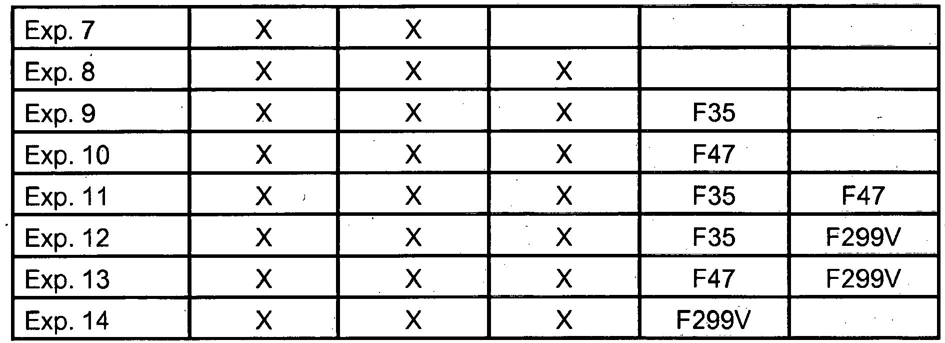

### Analysis of IL-10 and TNF-α in different Experiments:

With the results obtained in the first 5 experiments, it was proved that there exist no crossed interactions in the valuation methods with other elements used in the experimentation, that is, only TNF-α and IL-10 variations could be determined due to the presence of THP-1. The normalized slope between the obtained values at 6 and 24 hours was null for experiments 1-5 (without THP), thus it can be deduced that there are no crossed reactions.

In TABLE 13 the values of IL-10 and TNF-α obtained for experiments 6 to 14 (Exp. 6-Exp. 14) are shown.

**TABLE 13. Analysis of immunomodulation ability of different probiotics and mixtures thereof.**

| Exp. | 24 h | | 6h | | (24-6 h) | | Slope | | Normalized Slope | |
|---|---|---|---|---|---|---|---|---|---|---|
| | IL-10 (pg/ml) | TNF-α (pg/ml) | IL-10 (pg/ml) | TNF-α (pg/ml) | IL-10 (pg/ml) | TNF-α (pg/ml) | IL-10 (pg/ml) | TNF-α (pg/ml) | IL-10 (pg/ml) | TNF-α (pg/ml) |
| Exp. 6 | 0.945 | 1.730 | 0.805 | 1.675 | 0.14 | 0.05 | 0.01 | 0.002 | 0.72 | 0.14 |
| Exp. 7 | 1.045 | 1.830 | 0.915 | 1.675 | 0.13 | 0.16 | 0.01 | 0.01 | 0.59 | 0.39 |
| Exp. 8 | 33.055 | 5613.53 | 18.02 | 5980.46 | 15-04 | -366.03 | 0.63 | -15.29 | 3.48 | -0.26 |
| Exp. 9 | 234.505 | 2042.395 | 27.755 | 3701.56 | 206.75 | -1650.17 | 8.61 | -69.13 | 31.04 | -1.87 |
| Exp. 10 | 283.70 | 2380.045 | 31.365 | 3927.345 | 252.34 | -1547.30 | 10.51 | -64.47 | 33.52 | -1.64 |
| Exp. 11 | 348.28 | 1054.155 | 28.11 | 3820.925 | 320.17 | -2766.77 | 13.34 | -115.28 | 47.46 | -3.02 |
| Exp. 12 | 423.94 | 3411.85 | 45.475 | 4605.655 | 378.47 | -1193.81 | 15.77 | -49.74 | 34.68 | -1.08 |
| Exp. 13 | 270.805 | 3175.83 | 32.25 | 4420.09 | 238.56 | -1244.26 | 9.94 | -51.84 | 30.82 | -1.17 |
| Exp. 14 | 232.93 | 4111.85 | 37.925 | 4693.815 | 195.01 | -581.06 | 8.13 | -24.25 | 21.42 | -0.52 |

Previously to proceed with the interpretation of the results for Exp. 6 to Exp.14, it is necessary to emphasize the values obtained in experiments 6, 7 and 8. In both first ones, it is possible to observe that IL-10 and TNF-α production is low in THP-1 cells without LPS not probiotic strains (with or without antibiotic presence; Exp.6 - Exp.7). This is an important fact for the interpretation of normalized slopes calculated in the experiments, since medium or high production of interleukins by THP-1 cells by an inherent way could hide the obtained experimental values.

The obtained values in experiment 8 (Exp.8), in relation to the negative value obtained in the normalized slope of TNF-α production, is attributed to feedback negative phenomena, very common in the biologic systems. That is, the elevated production of pro-inflammatory interleukins by THP-1 cells leads them to an autoregulation of TNF-α production. Nevertheless, it is necessary to emphasize that, in spite of a reduction in the concentration of TNF-α production at 24 hours, values at 6 and 24 hours in this Exp. 8 are the most elevated of all the experimental series. In this Exp. 8 the value for IL-10 are lower than those obtained when THP-1 cells are inducted by LPS and in the presence of probiotic strains. In a net way, it can be deduced that the induction with LPS leads to an inflammatory response in THP-1 cells.

Based on these first premises, it is possible to realize that the experiment 11 (Exp.11) is the one in which a higher induction of IL-10 production (between 6 and 24 hours) and a higher reduction of TNF-α production is observed. In Exp.11 the cells were contacted with the probiotic Lp 3547 (F35 + F47), after an LPS induction of THP-1.

When observing the values obtained in experiments with strains F35 and F47 independently added, the results show that there exists cooperative function when both strains remain together (probiotic Lp 3547), since values, when they are individually added, in spite of being good ones, do not achieve at the values obtained when they are used together. This cooperate function is not detected when each of them (F35 or F47) are in contact with THP-1 cells and with Lactobacillus plantarum 299V (Exp.12 and Exp.13). Although the values obtained in experiments 12 (Exp. 12) and 13 (Exp. 13) are interesting ones, they are fast lower than those obtained in experiment 11 (Exp. 11).

The obtained values in experiment 14, that correspond to the effect of strain L299V when used individually, reinforce the idea of the immunomodulation superiority of the strains F35 and F47 (Exp. 9 and Exp.10) individually and, specially, when they are used together (Exp.11) in the Lp 3547 probiotic form. Indeed, from the results of TABLE 13 is clearly derived that the effect of the two strains of Lactobacillus plantarum (F35 and F47) together (Lp3547) is higher than the effect obtained by each strain independently, or when combined with Lactobacillus plantarum 299V, or when only L299V is added to the medium. Consequently, it can be concluded that the use of the two strains together, implies a synergy in the response of THP-1 cells to the LPS induction.

The observed reduction in the inflammatory response induced by the presence of LPS and other toxins at intestinal level has a direct effect at systemic level. The effect over the systemic level avoids any immunological deregulations, or the prosecution of a long term inflammatory state, which usually lead to chronic inflammatory states with implication in serious diseases like autoimmune reactions or intestinal injuries such as the Crohn's Syndrome.

It has to be emphasized that the described effects have been demonstrated using the strains in a viable form and without any alteration (sonication, fraction purification, etc.). This viable form mimesis the way in which the probiotic would be administered to a mammal, including humans. This is an important fact, since we can find studies with Lactobacillus plantarum 299V, in which the ability of this strain to cause immunomodulation by means of the reduction of TNF-α expression is assayed with extracts of L299V, being the extracts obtained from bacterial pellet sonication. This represents an unreal situation, since although it is perfectly acceptable that is a loss of a percentage viability of microorganisms during passage by gastrointestinal tract; this loss implies neither a rupture of the cell nor disintegration as that derived from a sonication process.

Thus, the experiments with the viable form of the strains showed in this section 11 represent a more suitable model to study the effect of the same, and allow the extrapolation to a real administration pattern in mammals, including humans.

## Claims

1. A strain of Lactobacillus plantarum deposited in the Colección Española de Cultivos Tipo (CECT) under the accession number CECT 7315.

2. A strain of Lactobacillus plantarum deposited in the Coleccion Española de Cultivos Tipo (CECT) under the accession number CECT 7316.

3. A bacterial culture which comprises the strain according to claim 1 or the strain according to claim 2 or a mixture thereof.

4. A product which comprises an effective amount of the strain as defined in claim 1 or of the strain as defined in claim 2 or of a mixture thereof.

5. An edible product which comprises an effective amount of the strain as defined in claim 1 or of the strain as defined in claim 2 or of a mixture thereof, together with appropriate amounts of other edible ingredients.

6. The edible product according to claim 5, which is selected, from the group consisting of a milk product, a yogurt, a curd, a cheese, a fermented milk, a milk powder, a milk based fermented product, an ice-cream, a fermented cereal based product, a milk based powder, a beverage, a dressing, a pet food, a dietary supplement and an infant formula.

7. A pharmaceutical composition which comprises a therapeutically effective amount of the strain as defined in claim 1 or of the strain as defined in claim 2 or of a mixture thereof, together with appropriate amounts of pharmaceutical acceptable excipients and/or carriers.

8. The strain as defined in any of the claims 1-2, for use in the food industry.

9. The strain as defined in any of the claims 1-2, for use as a probiotic.

10. A mixture of the strains as defined in claim 1 and 2, for use in the food industry.

11. A mixture of the strains as defined in claim 1 and 2, for use as a probiotic.

12. Use of the strain as defined in any of the claims 1-2, for the manufacture of an edible product.

13. Use of a mixture of the strains as defined in claim 1 and 2, for the manufacture of an edible product.

14. A strain as defined in any of the claims 1-2, for use as a medicament enhancing the immune system of an animal including a human.

15. A mixture of the strains as defined in any of the claim and 2, for use as a medicament enhancing the immune system of an animal including a human.

16. A process for the preparation of an edible product, comprising cultivating the strain as defined in claim 1 or the strain as defined in claim 2 or a mixture thereof, in a suitable medium.

## Patentansprüche

1. Ein Stamm von Lactobacillus plantarum, der bei der Colección Española de Cultivos Tipo (CECT) unter der Hinterlegungsnummer CECT 7315 hinterlegt ist.

2. Ein Stamm von Lactobacillus plantarum, der bei der Colección Española de Cultivos Tipo (CECT) unter der Hinterlegungsnummer CECT 7316 hinterlegt ist.

3. Eine Bakterienkultur, die den Stamm nach Anspruch 1 oder den Stamm nach Anspruch 2 oder eine Mischung davon umfasst.

4. Ein Produkt, das eine wirksame Menge des Stamms wie in Anspruch 1 definiert oder des Stamms wie in Anspruch 2 definiert oder einer Mischung davon umfasst.

5. Ein essbares Produkt, das eine wirksame Menge des Stamms wie in Anspruch 1 definiert oder des Stamms wie in Anspruch 2 definiert oder einer Mischung davon, zusammen mit geeigneten Mengen anderer essbaren Zutaten umfasst.

6. Das essbare Produkt nach Anspruch 5, das ausgewählt ist aus der Gruppe bestehend aus einem Milchprodukt, einem Joghurt, einem Käsebruch; einer Käse, einer fermentierten Milch; einem Milchpulver, einem auf Milch basierenden fermentierten Produkt, einem Speiseeis, einem auf Getreide basierenden fermentierten Produkt, einem auf Milch basierenden Pulver, einem Getränk, einem Dressing, einem Tierfutter, einem Nahrungsergänzungsmittel und einer Säuglingsnahrung.

7. Eine pharmazeutische Zusammensetzung umfassend eine therapeutisch wirksame Menge des Stamms wie in Anspruch 1 definiert oder des Stamms wie in Anspruch 2 definiert oder einer Mischung davon, zusammen mit angemessenen Mengen von pharmazeutisch akzeptablen Hilfsstoffen und/oder Arzneimittelträgern.

8. Der Stamm wie in einem der Ansprüche 1-2 definiert zur Verwendung in der Nahrungsmittelindustrie.

9. Der Stamm wie in einem der Ansprüche 1-2 definiert, zur Verwendung als probiotisches Mittel.

10. Eine Mischung von den Stämmen wie in Anspruch 1 und 2 definiert zur Verwendung in der Nahrungsmittelindustrie.

11. Eine Mischung von den Stämmen wie in Anspruch 1 und 2 definiert, zur Verwendung als probiotisches Mittel.

12. Verwendung des Stamms wie in einem der Ansprüche 1-2 definiert, für die Herstellung eines essbaren Produkts.

13. Verwendung einer Mischung von den Stämmen wie in Anspruch 1 und 2 definiert, für die Herstellung eines essbaren Produkts.

14. Ein Stamm wie in einem der Ansprüche 1-2 definiert, zur Verwendung als Arzneimittel zur Förderung des Immunsystems eines Tieres, einschließlich eines Menschen.

15. Eine Mischung von den Stämmen wie in einem der Ansprüche 1 und 2 definiert, zur Verwendung als Arzneimittel zur Förderung des Immunsystems eines Tieres, einschließlich eines Menschen.

16. Ein Verfahren für die Herstellung eines essbaren Produkts, umfassend die Kultur des Stamms wie in Anspruch 1 definiert oder des Stamms wie in Anspruch 2 definiert oder einer Mischung davon in einem geeigneten Medium.

## Revendications

1. Une souche de Lactobacillus plantarum déposée à la Colección Española de Cultivos Tipo (CECT) sous le numéro de dépôt CECT 7315.

2. Une souche de Lactobacillus plantarum déposée à la Colección Española de Cultivos Tipo (CECT) sous le numéro de dépôt CECT 7316.

3. Une culture bactérienne qui comprend la souche selon la revendication 1 ou la souche selon la revendication 2 ou un mélange de celles-ci.

4. Un produit qui comprend une quantité efficace de la souche telle que définie dans la revendication 1 ou de la souche telle que définie dans la revendication 2 ou d'un mélange de celles-ci.

5. Un produit comestible qui comprend une quantité efficace de la souche telle que définie dans la revendication 1, de la souche telle que définie dans la revendication 2 ou d'un mélange de celles-ci, avec des quantités appropriées d'autres ingrédients comestibles.

6. Le produit comestible selon la revendication 5, qui est choisi dans le groupe constitué d'un produit laitier, un yaourt, un fromage caillé ; un fromage, un lait fermenté ; une poudre de lait, un produit fermenté à base de lait, une crème glacée, un produit fermenté à base de céréale, une poudre à base de lait, une boisson, un assaisonnement, un aliment pour animaux de compagnie, un complément alimentaire et une préparation pour nourrissons.

7. Une composition pharmaceutique qui comprend une quantité thérapeutiquement efficace de la souche telle que définie dans la revendication 1 ou de la souche telle que définie dans la revendication 2 ou d'un mélange de celles-ci, avec des quantités appropriées d'excipients et/ou de véhicules pharmaceutiques acceptables.

8. La souche telle que définie dans l'une quelconque des revendications 1-2, pour l'utilisation dans l'industrie alimentaire.

9. La souche telle que définie dans l'une quelconque des revendications 1-2, pour l'utilisation en tant que matériau probiotique.

10. Un mélange des souches telles que définies dans la revendication 1 et 2, pour l'utilisation dans l'industrie alimentaire.

11. Un mélange des souches telles que définies dans la revendication 1 et 2, pour l'utilisation en tant que matériau probiotique.

12. L'utilisation de la souche telle que définie dans l'une quelconque des revendications 1-2, pour la manufacture d'un produit comestible.

13. Utilisation d'un mélange des souches telles que définies dans la revendication 1 et 2 pour la manufacture d'un produit comestible.

14. Une souche telle que définie dans l'une quelconque des revendications 1-2, pour l'utilisation en tant que médicament renfonçant le système immunitaire d'un animal, y compris les humains.

15. Un mélange des souches telles que définies dans l'une quelconque des revendications 1 et 2, pour l'utilisation en tant que médicament renfonçant le système immunitaire d'un animal, y compris les humains.

16. Un procédé pour la préparation d'un produit comestible, comprenant cultiver la souche telle que définie dans la revendication 1 ou la souche telle que définie dans la revendication 2, ou un mélange de celles-ci, dans un milieu approprié.


### SEQUENCE LISTING

<110> CARINSA. Creaciones Aromáticas Industriales SA.
<120> Strains of Lactobacillus plantarum as probiotics with immunomodulatory specific effect
<130> P1049PC00
<150> EP 07121817.6
   <151> 2007-11-29
<160> 6
<170> PatentIn version 3.5
<210> 1
   <211> 19
   <212> DNA
   <213> Artificial
<220>
   <223> Eub27f, forward primer for amplification of 16S gene
<400> 1
   gagtttgatc ctggctcag 19
<210> 2
   <211> 22
   <212> DNA
   <213> Artificial
<220>
   <223> Eub1492r, reverse primer for amplification of 16S gene
<400> 2
   tacggytacc ttgttacgac tt 22
<210> 3
   <211> 20
   <212> DNA
   <213> Artificial
<220>
   <223> forward primer 27f for sequencing 16S gene
<400> 3
   agagtttgat cctggctcag 20
<210> 4
   <211> 57
   <212> DNA
   <213> Artificial
<220>
   <223> forward primer 357f for sequencing 16S gene
<400> 4
   cgcccgccgc gccccgcgcc cggcccgccg cccccgcccc cctacgggag gcagcag 57
<210> > 5
   <211> 20
   <212> DNA
   <213> Artificial
<220>
   <223> reverse primer 907r for sequencing 16S gene
<400> 5
   ccgtcaattc ctttgagttt 20
<210> 6
   <211> 19
   <212> DNA
   <213> Artificial
<220>
   <223> reverse primer 1492r for sequencing 16S gene
<400> 6
   ggttaccttg ttacgactt 19